# EUROPEAN PATENT SPECIFICATION

(11) **EP 3 300 796 B1**
(45) Date of publication and mention of the grant of the patent: **07.06.2023**
(21) Application number: 17202343.4
(22) Date of filing: 02.09.2015
(51) Int. Cl.: B01J 19/00, C07C 253/18, C07C 253/34, C07C 255/08, B01D 1/00, B01D 1/26, B01D 3/14

(54) **EVAPORATION SYSTEM FOR A PROCESS STREAM**
VERDAMPFUNGSSYSTEM FÜR EINEN PROZESSSTROM
SYSTÈME D'ÉVAPORATION POUR UN FLUX DE TRAITEMENT

(30) Priority: 29.09.2014 CN 201410511314
(43) Date of publication of application: 04.04.2018
(62) Divisional of application: 15760075.0
(73) Proprietor: Ineos Europe AG, 1180 Rolle (Vaud) (CH)
(72) Inventor: MCDONEL, Timothy, Elburn, IL 60119 (US); COUCH, Jay, Naperville, IL 60564 (US); WAGNER, David, Naperville, IL 60564 (US); WACHTENDORF, Paul, Victoria, TX 77904 (US)
(74) Representative: King, Alex

(56) References cited:
- CA-A- 1 164 014
- CN-A- 102 657 946
- US-A- 4 166 008
- US-A- 4 334 965

## Description

### FIELD OF THE INVENTION

An evaporation process for removing heavy organic impurities from a process stream is provided. More specifically, the process includes providing a bottoms stream from an extractive distillation column, and separating water from heavy organics from the bottoms stream in an evaporator system. The evaporator system is effective for providing an aqueous condensate and liquid residue.

### BACKGROUND

Various processes and systems for the manufacture of acrylonitrile and methacrylonitrile are known; see for example, U.S. Patent No. 6,107,509. Typically, recovery and purification of acrylonitrile/methacrylonitrile produced by the direct reaction of a hydrocarbon selected from the group consisting of propane, propylene or isobutylene, ammonia and oxygen in the presence of a catalyst has been accomplished by transporting the reactor effluent containing acrylonitrile/methacrylonitrile to a first column (quench) where the reactor effluent is cooled with a first aqueous stream, transporting the cooled effluent containing acrylonitrile/methacrylonitrile into a second column (absorber) where the cooled effluent is contacted with a second aqueous stream to absorb the acrylonitrile/methacrylonitrile into the second aqueous stream, transporting the second aqueous stream containing the acrylonitrile/methacrylonitrile from the second column to a first distillation column (recovery column) for separation of the crude acrylonitrile/methacrylonitrile from the second aqueous stream, and transporting the separated crude acrylonitrile/methacrylonitrile to a second distillation column (heads column) to remove at least some impurities from the crude acrylonitrile/ methacrylonitrile, and transporting the partially purified acrylonitrile/methacrylonitrile to a third distillation column (product column) to obtain product acrylonitrile/methacrylonitrile. U.S. Pat. Nos. 4,234,510; 3,885,928; 3,352,764; 3,198,750 and 3,044,966 are illustrative of typical recovery and purification processes for acrylonitrile and methacrylonitrile.

A process for recovery of olefinic nitriles is described in U.S. Pat. No. 4,334,965. As described in U.S. Pat. No. 4,334,965, a multi-stage evaporator is used to remove water from the extracted distillation or stripper tower bottoms recycled as quench liquid to the quench tower of an acrylonitrile purification and recovery system. This process results in a significant decrease in the amount of waste quench tower bottoms produced by the system. Use of a multi-effect evaporator represents a significant energy savings as compared with other techniques for decreasing the water content of the recycle stream. U.S. Pat. No. 4,334,965 discloses that by means of a multi-effect evaporator, 50% or more of the liquid in the recycled stream can be removed therefrom leaving a concentrated recycle stream to serve as the quench liquid in the same way as shown in U.S. Pat. No. 4,166,008. U.S. Pat. No. 4,334,965 discloses that because multi-effect evaporators are so energy efficient, however, the overall energy costs of this technique are much lower than the technique described in U.S. Pat. No. 4,166,008.

While the manufacture of acrylonitrile/methacrylonitrile has been commercially practiced for years there are still areas in which improvement would have a substantial benefit. One of those areas of improvement would be more efficient evaporator operation for recovery column bottoms.

### SUMMARY

Accordingly, an aspect of the disclosure is to provide a safe, effective and cost effective process that overcomes or reduces the disadvantages of conventional processes.

The evaporation process comprises conveying a bottoms stream from an extractive distillation column to an evaporator system, the evaporator system including one or more evaporation stages, wherein a first evaporation stage is configured to receive the bottoms stream from the distillation column, providing an aqueous condensate and a liquid residue from the one or more evaporation stages, wherein the evaporation percentage of the bottoms of the extractive distillation column is greater than 60% and wherein the aqueous condensate has 0.1 weight percent or less heavy organic impurities and the liquid residue has 3 to 10 wt% heavy organic impurities; and conveying the aqueous condensate to a quench column and/or light organic stripper.

The above and other aspects, features and advantages of the present disclosure will be apparent from the following detailed description of the illustrated embodiments thereof which are to be read in connection with the accompanying drawings.

### BRIEF DESCRIPTION OF THE DRAWINGS

A more complete understanding of the exemplary embodiments of the present disclosure and the advantages thereof may be acquired by referring to the following description in consideration of the accompanying drawing in which like reference numbers indicate like features and wherein:
FIG. 1 is a schematic flow diagram of a process for the manufacture of acrylonitrile product.
FIG. 2 is a schematic flow diagram of an alternative process for the manufacture of acrylonitrile product.

### DETAILED DESCRIPTION

### Ammoxidation Process

In one aspect, a process stream is provided from an ammoxidation reaction process. An example of one such process is described in U.S. Pat. No. 4,334,965.

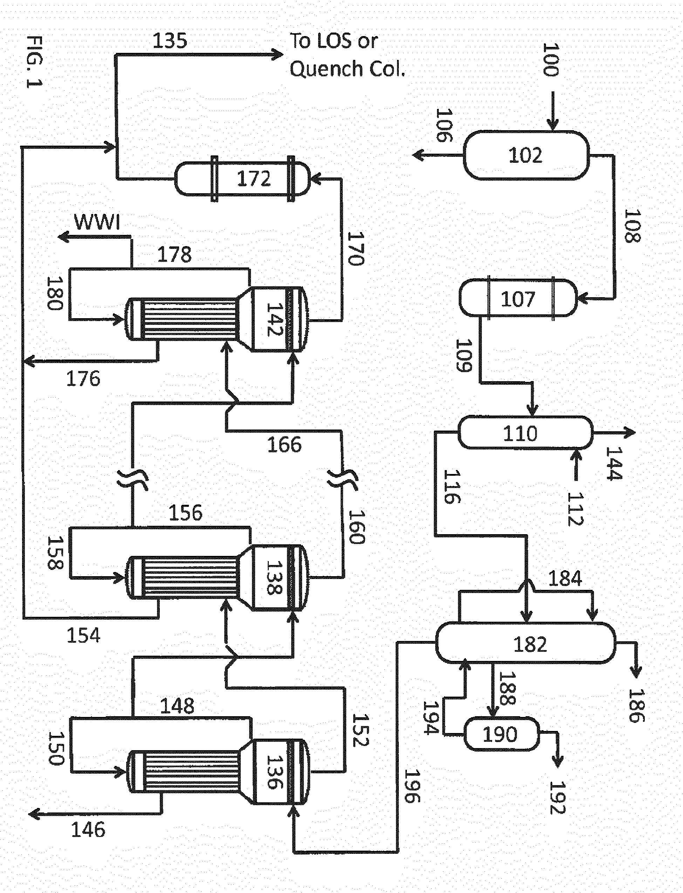

Figure 1 is an overall schematic representation of various aspects. Referring to FIG. 1, the reactor effluent gas in conduit 100 comprising acrylonitrile, HCN, acetonitrile, water vapor and impurities may be first passed to a quench column 102. The gas may be contacted with quench liquid in quench column 102. A bottoms stream containing water and impurities may be removed through conduit 106 and sent to waste treatment.

The cooled reactor effluent gases may leave the quench system through line 108 and pass to quench aftercooler 107. The quench aftercooler 107 is effective for cooling the quench effluent to below about 50 °C. Cooled quench effluent is feed through line 109 to absorber 110. Wash water may enter absorber 110 at the top through line 112. Non-condensable gases may be removed from the absorber through line 114. An aqueous solution containing water, acrylonitrile, acetonitrile and impurities may be removed as a bottoms stream through line 116 and passed to extractive distillation column 182.

Solvent water may be introduced to the top of extractive distillation column 182 through line 184 to perform extractive distillation. Acrylonitrile and HCN may be removed as an overhead vapor through line 186 and sent to further purification (not shown). A stream containing acetonitrile and water may be removed through line 188 and passed to stripper 190. Heat may be added to stripper 190 to remove acetonitrile as an overhead vapor through line 192. The bottoms stream containing water, heavy organics and other impurities may be removed from extractive distillation column 182 through line 196. A liquid stream containing mostly water may be removed from the lower half of stripper 190 through line 194 and used as solvent water to extractive distillation column 182.

### Evaporator System

Extractive distillation column bottoms in line 196, which may also be referred to as a process stream, is subjected to evaporation in an evaporator system. In this aspect, the extractive distillation column bottoms in line 196 that may enter heat exchanger 136 comprises water, polymer, ammonia, and acrylonitrile. As used herein, "heavy organic impurities" refers to polymer. As used herein polymer refers to a mixture of heavy organic material and a slight amount of light organics. Heavy organic material may include a mixture of different high boiling organic compounds having a high degree of nitrile substitution and also containing some oxygenated hydrocarbon groups. In this aspect, the process stream includes about 0.5 to about 1.5 weight percent heavy organic impurities, and in another aspect, about 0.75 to about 1.25 weight percent.

The evaporator system includes one or more evaporation stages effective for providing an aqueous condensate and liquid residue. For example, the evaporator system may include 1 to about 6 evaporation stages, in another aspect, 2 to about 6 evaporation stages, in another aspect, 2 to about 5 evaporation stages, in another aspect, 2 to about 4 evaporation stages, and in another aspect, 2 to about 3 evaporation stages.

In one aspect illustrated in Figure 1, an evaporator system includes shell and tube heat exchangers 136, 138 and 142 arranged in series. As used herein, an "evaporation stage" refers to a single heat exchanger. In each heat exchanger, liquid in the tube side of the exchanger is partially evaporated producing a vaporous effluent and a liquid effluent. The liquid effluent is fed to the tube side of the next heat exchanger in the series while the vaporous effluent is fed to the shell side of the same heat exchanger causing additional partial evaporation of the liquid. This technique is continued for as many stages as is necessary to remove the desired amount of water from the stripper bottoms. In each stage, condensate produced when the heat-supplying vapor is condensed through heat exchange is recovered and either recycled for reuse or subjected to chemical or biological purification.

Bottoms stream containing water, heavy organics and other impurities may be removed from extractive distillation column 182 through line 196 and passed into the tube side of first heat exchanger 136, while low pressure steam is passed through the shell side of this heat exchanger. In one aspect, a flow through the tube side of the heat exchanger is about 1 to about 3 meters/second, and in another aspect, about 1.5 to about 2.5 meters/second. Heat exchange therein causes the lower pressure steam to condense and the extractive distillation column bottoms to partially evaporate. Condensate may be removed from first heat exchanger 136 for reuse via line 146.

Heating of the extractive distillation column bottoms in first heat exchanger 136 causes partial separation thereof into vapor and liquid phases. In aspects where more than one heat exchanger is used, the liquid phase is withdrawn via line 148 and transferred to the tube side of second heat exchanger 138, a portion of the withdrawn liquid being recycled via line 150 to the bottom of the tube side of first heat exchanger 136. Vapor produced in first heat exchanger 136 is withdrawn and transferred via line 152 to the shell side of second heat exchanger 138. Heat exchange in heat exchanger 138 causes condensation of the vapor on the shell side and partial evaporation of the liquid in the tube side, thereby producing liquid in vapor phases in second heat exchanger 138. Condensate produced on the shell side of second heat exchanger 138 is discharged via line 154. This condensate has a relatively low concentration of heavy organics such as polymer and the like.

In aspects, where more than two heat exchangers are used, the liquid phase remaining in the tube side of second heat exchanger 138 is transferred via line 156 to the tube side of third heat exchanger 142, a portion of the liquid being recycled via line 158 to the tube side of second heat exchanger 138. Vapor produced in the tube side of second heat exchanger 138 is transferred via line 160 to the shell side of third heat exchanger 142. Again, heat exchange in third heat exchanger 142 causes condensation of the vapor on the shell side to form a condensate which is withdrawn via line 176 and conveyed in the same way as the condensate from second heat exchanger 138.

The vapor produced in the tube side of third heat exchanger 142 is withdrawn via line 170, condensed in condenser 172, and recovered in a utility water vessel and /or combined with condensate from lines 146, 154, 162, and/or 176. The condensate from the shell side of heat exchanger 142 may also be transferred to the utility water vessel via line 176, for example, as combined and provided at 135. Liquid recovered from the tube side of third heat exchanger 142 may be withdrawn via line 178 and recycled via line 180 to the tube side of the third heat exchanger. Aqueous condensates may be of such high purity that they may be used as conventional clean water such as, for example, in the flushing of various process equipment, as a return to the quench column (for example a first stage of a quench column), and/or to the light organic stripper. In particular, the aqueous condensate has about 0.1 weight percent or less heavy organic impurities, in another aspect, about 0.075 heavy organic impurities, in another aspect, about 0.05 heavy organic impurities, and in another aspect, about 0.025 heavy organic impurities.

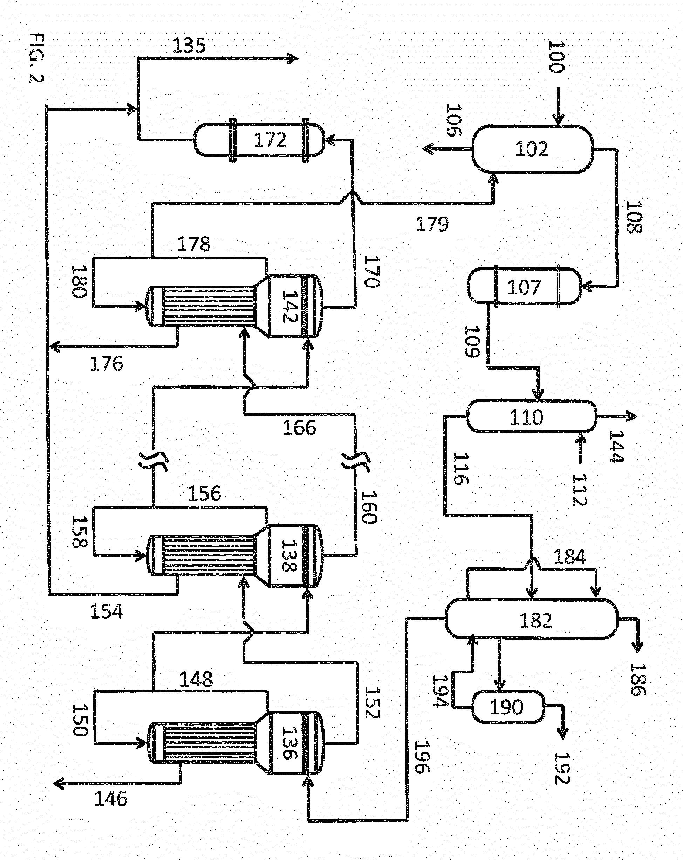

The liquid residue has 3 to 10 weight percent heavy organic impurities, in another aspect, 4 to 8 weight percent heavy organic impurities, and in another aspect, 5 to 7 weight percent heavy organic impurities. As shown in Figure 1, liquid residue may be sent to a waste water incinerator (WWI). Alternatively, as shown in Figure 2, the liquid residue may be sent via line 179 to a lower portion of a quench tower 102.

In one aspect, the aqueous condensates produced in the second and third heat exchangers contain extremely small amounts of heavy organics. Thus, they can be directly processed by conventional biological or chemical treatment to produce environmentally acceptable water. Furthermore, the condensate produced in the fourth heat exchanger as well as the condensate produced by the condenser are pure enough to be used for various process purposes such as wash water without further treatment. The condensate produced by the first heat exchanger, since it does not contact any other process stream, is highly pure.

### Evaporation Percentage

In one aspect, higher evaporation may be beneficial because the evaporator condensate may be reused or disposed of while the liquid residue from the last stage of the multi-stage evaporator may be incinerated and/or otherwise disposed of.

Disclosed herein is also that the evaporation percentage of the bottoms of extractive distillation column may be greater than about 55% to about 85%. In the present invention, the evaporation percentage of the bottoms of extractive distillation column is greater than 60%. In an aspect, the evaporation percentage of the bottoms of extractive distillation column may be greater than about 60% to about 85%. In an aspect, the evaporation percentage of the bottoms of extractive distillation column may be in the range of about 73% to about 75%.

By running a four (4) stage evaporation process with about a 60-65%, in one aspect, about 63% vaporization percentage, the percent of liquid polymer in the evaporator bottoms coming out of the fourth and last heat exchanger 142 may be about 3% by weight.

By running a four (4) stage evaporation process with about a 80-85%, in one aspect about 83% vaporization percentage, the percent of liquid polymer in the evaporator bottoms coming out of the fourth and last heat exchanger 142 may be about 6% by weight.

By running a four (4) stage evaporation process with about a 73-75%, in one aspect about 74% vaporization percentage, the percent of liquid polymer in the evaporator bottoms coming out of the fourth and last heat exchanger 142 may be about 5.5% by weight.

In an aspect, each evaporative stage provides an evaporation rate of about 15 to 25%.

In another aspect, the amount of polymer in the liquid residue is about 3% by weight. In an aspect, the ratio of the percent evaporation percentage to percent by weight of polymer is about 60-65:3.

In an aspect, the evaporation percent is about 82 to about 83%, and the amount of polymer in the liquid residue is about 6.0% by weight. The condensate is fed as feed to a light organic stripper LOS (not shown) via line 135 for further processing, or sent to quench column 102 as quench liquid. In an aspect, the ratio of the percent evaporation percent to percent by weight of polymer is about 82-83:6.

In an aspect, the evaporation percent is about 73 to about 75% and the polymer in the liquid residue is about 5.5% by weight. The condensate is fed as feed to a light organic stripper LOS (not shown) via line 135 for further processing, or sent to quench column 102 as quench liquid. In an aspect, the ratio of the percent evaporation percent to percent by weight of polymer is about 73-75:5.5.

In an aspect, it was found that when the evaporation percent is about 74%, substantially less fouling was experienced than at an evaporation percent of about 83%, while at the same time achieving a relatively high amount of polymer by weight in the liquid residue, i.e., 5.5% by weight, versus 6.0% by weight at the evaporation percent of about 83%. This is a surprising result, as it would have been expected that the amount of fouling would be linearly related to the percent by weight of polymer in the liquid stream.

It has been found that when the evaporation percent is greater than about 83%, there may be too much fouling (mostly on the tube side) in the fourth stage evaporator or heat exchanger 142. It has been found that an evaporation percent of about 73-75% significantly reduces the amount of fouling, while at the same time, providing a relatively high amount of polymer by weight in the liquid residue.

Any number of stages can be employed in the evaporator system. Furthermore, although low pressure steam is shown in the above description as supplying the heat necessary for all the evaporations, any heat source can be employed. In a typical acrylonitrile purifications and recovery plant, however, low pressure steam, that is saturated steam having a pressure of up to 689 kPa gauge (100 psig), normally about 138 to 414 kPag (about 20 to 60 psig), is readily available and is preferably used. Also, the amount of water in the stripper column removed by the multiple effect evaporator can be varied depending primarily upon economics. Finally, it should also be appreciated that the multiple effect evaporator of this disclosure need not be restricted to use on stripper column bottoms as shown in the above description, but can be employed to concentrate any other process stream which is recycled for use as the quench liquid. For example, a multi-effect evaporator can be used to process the extractive distillation tower bottoms recycled in line 156 of FIG. 2 of U.S. Pat. No. 4,166,008.

### Quench Column Operation

In one aspect, a process for operating a quench column includes conveying a reactor effluent to a quench column and contacting the reactor effluent with water containing polymer in an effluent extraction zone to provide an extracted effluent stream. The process further includes contacting the extracted effluent stream with sulfuric acid in an acid contact zone and removing a first stream to provide a first quench column stream having about 10 weight percent or less polymer. In one aspect, the process includes providing at least a portion of the water from an evaporator system. As described herein, the water may be an aqueous condensate and/or a liquid residue.

In one aspect, formula y = -M1x + C1 where y is a weight % of ammonium sulfate, x is a weight % polymer, M1 is 4.6 or less and C1 is 45 or less defines an amount of ammonium sulfate and an amount of polymer in the quench column bottoms stream. In an related aspect, M1 is 1.5 or less and C1 is 30 or less. In one aspect, the process provides a quench column bottoms streams having about 10 to about 25 weight % ammonium sulfate and less than about 5 weight % polymer, in another aspect, about 15 to about 21 weight % ammonium sulfate and less than about 5 weight % polymer. The quench column bottoms stream has a pH of about 4.5 to about 6.0.

In another aspect, the process includes removing a second stream to provide a second quench column stream having more than about 10 weight percent polymer and less than about 5 weight percent ammonium sulfate.

In another aspect, at least a portion of the second quench column stream is recycled to the effluent extraction zone. The extracted effluent stream is countercurrent to the sulfuric acid. In one aspect, a first quench column stream is removed above the effluent extraction zone. Adiabatic cooling may occur in the effluent extraction zone.

In an aspect, a process may comprise controlling the amount of makeup water conveyed to the quench column and/or controlling the amount of sulfuric acid conveyed to the quench column to obtain a concentration of ammonium sulfate in a quench column bottoms stream of about 10 to about 25% by weight. In an aspect, the process may comprise detecting the pH of the liquid bottoms of the quench column bottoms and controlling the flow of sulfuric acid to the quench column based on the detected pH of the liquid bottoms to obtain a quench column bottoms stream having a pH of about 4.5 - 6.0. In an aspect, the process may comprise determining the concentration of ammonium sulfate in the quench column bottoms stream based on the flow rate of sulfuric acid to the quench column and the flow rate of the quench column bottoms stream from or out of the quench column. In an aspect, the process may comprise adjusting the flow rate of sulfuric acid and/or makeup water conveyed to the quench column based upon the concentration of ammonium sulfate determined in the determining step to maintain the concentration of ammonium sulfate in the range of about 10 to about 25% by weight. By increasing the concentration of ammonium sulfate in the quench column bottoms stream to about 10 to about 25% by weight from the 5-10% by weight provided in a conventional process, less water needs to be removed from the quench columns bottom stream to obtain even higher concentrations of ammonium sulfate. It has been found that by increasing the concentration of ammonium sulfate in the quench column bottoms stream to about 10 to about 25% by weight, a sulfate condenser may be used to efficiently condense the quench column bottoms stream to about 35-40% by weight.

It should be understood that the features of the disclosure are susceptible to modification, alteration, changes or substitution without departing from the scope of the claims. For example, the dimensions, number, size and shape of the various components may be altered to fit specific applications. Accordingly, the specific embodiments illustrated and described herein are for illustrative purposes only.

## Claims

1. An evaporation process comprising:
conveying a bottoms stream from an extractive distillation column to an evaporator system, the evaporator system including one or more evaporation stages, wherein a first evaporation stage is configured to receive the bottoms stream from the distillation column,
providing an aqueous condensate and a liquid residue from the one or more evaporation stages, wherein the evaporation percentage of the bottoms of the extractive distillation column is greater than 60% and wherein the aqueous condensate has 0.1 weight percent or less heavy organic impurities and the liquid residue has 3 to 10 wt% heavy organic impurities; and
conveying the aqueous condensate to a quench column and/or light organic stripper.

2. The process of claim 1 wherein the liquid residue is conveyed to a waste water incinerator.

3. The process of claim 1 wherein liquid residue is conveyed to a lower portion of the quench column.

4. The process of claim 1 wherein the evaporator system includes 1 to 6 evaporation stages, and preferably wherein the evaporator system includes 2 to 6 evaporation stages.

5. The process of claim 1 wherein the evaporator system includes 2 to 5 evaporation stages, and preferably wherein the evaporator system includes 2 to 4 evaporation stages.

6. The process of claim 1 wherein the evaporator system includes 2 to 3 evaporation stages.

7. The process of claim 1 wherein the bottoms stream includes 0.5 to 1.5 weight percent heavy organic impurities, and preferably wherein the heavy organic impurities includes polymeric materials produced in an ammoxidation reaction process.

8. The process of claim 1 wherein the aqueous condensate has 0.075 weight percent or less heavy organic impurities.

9. The process of claim 8 wherein the aqueous condensate has 0.05 weight percent or less heavy organic impurities.

10. The process of claim 9 wherein the aqueous condensate has 0.025 weight percent or less heavy organic impurities.

11. The process of claim 1 wherein the liquid residue has 4 to 8 weight percent heavy organic impurities, and preferably wherein the liquid residue has 5 to 7 weight percent heavy organic impurities.

12. The process of claim 1 wherein the evaporator system includes at least one shell and tube heat exchanger.

13. The process of claim 12 wherein a flow through a tube side of the heat exchanger is 1 to 3 meters/second.

14. The process of claim 1 wherein each evaporative stage provides an evaporation rate of 15 to 25%.

## Patentansprüche

1. Verdampfungsprozess, umfassend:
Fördern eines Bodenstroms von einer Extraktivdestillationskolonne zu einem Verdampfersystem, wobei das Verdampfersystem eine oder mehrere Verdampfungsstufen beinhaltet, wobei eine erste Verdampfungsstufe konfiguriert ist, um den Bodenstrom von der Destillationskolonne aufzunehmen,
Bereitstellen eines wässrigen Kondensats und eines flüssigen Rückstands aus der einen oder den mehreren Verdampfungsstufen, wobei der Verdampfungsprozentsatz des Bodens der Extraktivdestillationskolonne größer als 60 % ist und wobei das wässrige Kondensat 0,1 Gewichtsprozent oder weniger schwere organische Verunreinigungen aufweist und der flüssige Rückstand 3 bis 10 Gew.-% schwere organische Verunreinigungen aufweist; und
Fördern des wässrigen Kondensats zu einer Abschreckkolonne und/oder einem leichten organischen Stripper.

2. Prozess nach Anspruch 1, wobei der flüssige Rückstand zu einer Abwasserverbrennungsanlage gefördert wird.

3. Prozess nach Anspruch 1, wobei flüssiger Rückstand zu einem unteren Teil der Abschreckkolonne gefördert wird.

4. Prozess nach Anspruch 1, wobei das Verdampfersystem 1 bis 6 Verdampfungsstufen beinhaltet und wobei bevorzugt das Verdampfersystem 2 bis 6 Verdampfungsstufen beinhaltet.

5. Prozess nach Anspruch 1, wobei das Verdampfersystem 2 bis 5 Verdampfungsstufen beinhaltet und wobei bevorzugt das Verdampfersystem 2 bis 4 Verdampfungsstufen beinhaltet.

6. Prozess nach Anspruch 1, wobei das Verdampfersystem 2 bis 3 Verdampfungsstufen beinhaltet.

7. Prozess nach Anspruch 1, wobei der Bodenstrom 0,5 bis 1,5 Gewichtsprozent schwere organische Verunreinigungen beinhaltet und wobei bevorzugt die schweren organischen Verunreinigungen polymere Materialien beinhalten, die in einem Ammoxidationsreaktionsprozess produziert werden.

8. Prozess nach Anspruch 1, wobei das wässrige Kondensat 0,075 Gewichtsprozent oder weniger schwere organische Verunreinigungen aufweist.

9. Prozess nach Anspruch 8, wobei das wässrige Kondensat 0,05 Gewichtsprozent oder weniger schwere organische Verunreinigungen aufweist.

10. Prozess nach Anspruch 9, wobei das wässrige Kondensat 0,025 Gewichtsprozent oder weniger schwere organische Verunreinigungen aufweist.

11. Prozess nach Anspruch 1, wobei der flüssige Rückstand 4 bis 8 Gewichtsprozent schwere organische Verunreinigungen aufweist und wobei bevorzugt der flüssige Rückstand 5 bis 7 Gewichtsprozent schwere organische Verunreinigungen aufweist.

12. Prozess nach Anspruch 1, wobei das Verdampfersystem mindestens einen Rohrbündelwärmetauscher beinhaltet.

13. Prozess nach Anspruch 12, wobei eine Strömung durch eine Rohrseite des Wärmetauschers 1 bis 3 Meter/Sekunde beträgt.

14. Prozess nach Anspruch 1, wobei jede Verdampfungsstufe eine Verdampfungsrate von 15 bis 25 % bereitstellt.

## Revendications

1. Procédé d'évaporation comprenant :
l'acheminement d'un courant de queue d'une colonne de distillation extractive vers un système d'évaporateur, le système d'évaporateur comportant une ou plusieurs étapes d'évaporation, dans lequel une première étape d'évaporation est configurée pour recevoir le courant de queue de la colonne de distillation,
la fourniture d'un condensat aqueux et d'un résidu liquide provenant d'une ou plusieurs étapes d'évaporation, dans lequel le pourcentage d'évaporation des queues de la colonne de distillation extractive est supérieur à 60 % et dans lequel le condensat aqueux contient 0,1 % en poids ou moins d'impuretés organiques lourdes et le résidu liquide contient 3 à 10 % en poids d'impuretés organiques lourdes ; et
l'acheminement du condensat aqueux vers une colonne de trempe et/ou un extracteur organique léger.

2. Procédé selon la revendication 1, dans lequel le résidu liquide est acheminé vers un incinérateur d'eaux usées.

3. Procédé selon la revendication 1, dans lequel le résidu liquide est acheminé vers une partie inférieure de la colonne de trempe.

4. Procédé selon la revendication 1, dans lequel le système d'évaporateur comporte 1 à 6 étapes d'évaporation, et de préférence dans lequel le système d'évaporateur comporte 2 à 6 étapes d'évaporation.

5. Procédé selon la revendication 1, dans lequel le système d'évaporateur comporte 2 à 5 étapes d'évaporation, et de préférence dans lequel le système d'évaporateur comporte 2 à 4 étapes d'évaporation.

6. Procédé selon la revendication 1, dans lequel le système d'évaporateur comporte 2 à 3 étapes d'évaporation.

7. Procédé selon la revendication 1, dans lequel le courant de queue comporte 0,5 à 1,5 % en poids d'impuretés organiques lourdes, et de préférence dans lequel les impuretés organiques lourdes comportent des matériaux polymères produits dans un procédé de réaction d'ammoxydation.

8. Procédé selon la revendication 1, dans lequel le condensat aqueux contient 0,075 % en poids ou moins d'impuretés organiques lourdes.

9. Procédé selon la revendication 8, dans lequel le condensat aqueux contient 0,05 % en poids ou moins d'impuretés organiques lourdes.

10. Procédé selon la revendication 9, dans lequel le condensat aqueux contient 0,025 % en poids ou moins d'impuretés organiques lourdes.

11. Procédé selon la revendication 1, dans lequel le résidu liquide contient 4 à 8 % en poids d'impuretés organiques lourdes, et de préférence dans lequel le résidu liquide contient 5 à 7 % en poids d'impuretés organiques lourdes.

12. Procédé selon la revendication 1, dans lequel le système d'évaporateur comporte au moins un échangeur de chaleur à calandre et tube.

13. Procédé selon la revendication 12, dans lequel un débit à travers un côté tube de l'échangeur de chaleur est de 1 à 3 mètres/seconde.

14. Procédé selon la revendication 1, dans lequel chaque étape d'évaporation assure un taux d'évaporation de 15 à 25 %.
